# EUROPEAN PATENT APPLICATION

(11) **EP 4 299 068 A1**
(43) Date of publication of application: **03.01.2024**
(21) Application number: 22181324.9
(22) Date of filing: 27.06.2022
(51) Int. Cl.: A61K 36/63, A23L 19/00, A61P 43/00

(54) **METHOD FOR OBTAINING AN ENRICHED OLEACEIN EXTRACT FROM OLIVE LEAVES**

(71) Applicant: Robertet S.A., 06130 Grasse (FR)
(72) Inventor: SKALTSOUNIS, Alexios Leandros, 15127 Melissia, Athènes (GR); STATHOPOULOS, Panagiotis, 12243 Aigaleo, Athènes (GR); KOUTRA, Christina, 12135 Anthoupoli, Athènes (GR); HUMBERT, Marina, 06130 GRASSE (FR); PAPAEFSTATHIOU, Georgios, 16777 Elliniko, Athènes (GR)
(74) Representative: Axe PI

(57) **Abstract**

The present invention relates to a method for increasing the levels of oleacein in an olive tree leaves extract, wherein olive tree leaves are frozen and pre-treated by freeze drying before being extracted to obtain an olive tree leaves extract enriched in oleacein. The invention also relates to a composition comprising said olive tree leaves extract enriched in oleacein and its uses.

## Description

### FIELD OF THE INVENTION

The present invention relates to a new, rapid, economical, and eco-friendly method for obtaining oleacein-enriched extracts from alternative natural sources. The invention also relates to the uses of such oleacein-enriched extracts and compositions comprising the same.

### BACKGROUND

Olive tree is one of the main crops worldwide for olive oil and table olives production (Kashaninejad et al., 2020, Freeze dried extract from olive leaves: Valorisation, extraction kinetics and extract characterization. Food and Bioproducts Processing 124, 196-207). EU countries are the largest producer in the world, where more than 800 million olive trees are cultivated for olive oil as well as for table olives. During olive oil and table olive production, huge amounts of by-products are produced, such as the olive pomace, the mill wastewaters, the stones, and the leaves. The olive pomace is the main by-product generated and is a mixture of olive fruits after the recovery of olive oil, together with vegetation waters and stones.

However, 80% of this massive production becomes untreated biomass per year according to Food and Agricultural Organization (FAO STAT), raising huge environmental threats. This biomass is derived from harvest residues and olive tree pruning - thin branches (around 50% by weight), leaves (25%) and thick branches or wood (25%)-, as well as from olive mill by-products (removed leaves and thin branches from the olive fruit, olive oil pomace, olive stones, olive skins, olive mill wastewater) (Salido et al., 2015, Phenolic Components and Antioxidant Activity of Wood Extracts from 10 Main Spanish Olive Cultivars. J Agric Food Chem 63, 6493-6500).

Despite their harmful effects, olive tree by-products are a valuable source of bioactive compounds with proved biological properties (antioxidant, antitumor, anti-inflammatory, antimicrobial, cardioprotective, etc). Secoiridoids, flavonoids, lignans, triterpenic acids simple phenols are some of the main categories of bioactive compounds identified in these by products (Cavaca et al., 2020, Chapter 5 - The olive-tree leaves as a source of high-added value molecules: Oleuropein, in: Atta-Ur-Rahman (Ed.), Studies in Natural Products Chemistry, Bioactive Natural Products. Elsevier, pp. 131-180). In 2012, European Food Safety Authority (EFSA) approved a health claim on olive oil polyphenols, certifying their beneficial health properties.

The secoiridoids in the family of Oleaceae are derived from the oleoside type of glucosides. The phenolic class of secoiridoids included the most important components protecting olive oil against autoxidation processes; these include oleacein (dialdehydic from of decarboxymethyl oleuropein aglycon), oleocanthal (dialdehydic form of decarboxymethyl ligustrosde aglycon) and oleuropein.

One of the most important phenolic compounds is oleuropein, which is a major metabolite in olive leaves. Oleuropein is a secoiridoid, a hydroxytyrosol analogue linked with one monoterpene and one glucose unit. There is large commercial interest, since due to its antioxidant and anti-inflammatory effects; oleuropein presents also neuroprotective, cardioprotective, antitumor and antidiabetic activity. Much research has been done on oleuropein's quantitative recovery from olive leaves and the impact of drying procedures and extraction conditions; however, the results are contradictory. There is no integrated study on all these crucial parameters that affect oleuropein's content in extracts, neither a specific protocol suggests the optimum process to produce oleuropein enriched extracts (Talhaoui et al., 2015, Phenolic compounds in olive leaves: Analytical determination, biotic and abiotic influence, and health benefits. Food Research International, FOOD BIOACTIVE COMPOUNDS: QUALITY CONTROL AND BIOACTIVITY 77, 92-108).

Oleacein (OLEA) is another phenolic compound with great scientific and commercial interest for pharmaceutical, food and cosmetics industry. Oleacein is major metabolite in olive oil but is scarcely found in olive leaves and it is structurally very similar with oleuropein, composed of one hydroxytyrosol unit linked with decarboxymethyl elenolic acid. It is a natural product derived from oleuropein's enzymatic biotransformation induced by endogenous enzymes (esterase, b-glucosidase, polyphenoloxidase, peroxidase) activated during olive fruit maturation, crush and malaxation.

Among oleacein's pharmacological properties some of the most important are free radical scavenging, blood pressure, inflammation regulation and its protective effects against specific types of cancer.

The only natural source of oleacein until now, is extra virgin olive oil (EVOO). However, oleacein's concentration in EVOO is mutable depending on many factors, such as the variety of olive trees, the cultivation process, the kind of soil and climate conditions, the maturation level of the olive fruit and the conditions applied during olive oil extraction process. Moreover, the limited half - life span due to the activity of hydrolytic enzymes and the restricted stability induced by oxidation are crucial factors that decrease oleacein's content in the final product.

It is well-known that the olive fruit composition can be strongly affected by agronomic factors as genetics, cultivar, ripening stage, environmental growth conditions including biotic and abiotic stresses.

Harvest period principally influenced oleacein, oleocanthal, oleuropein and ligustroside aglycones and flavonoids.

The biosynthesis of oleuropein is particularly affected by the olive fruit ripening process and the season.

In general, during fruit ripening, there is a decrease in the amount of polar phenolic and alpha tocopherol.

The oleocanthal and oleacein concentration has been positively correlated with an early harvest of olives. In the green stage, the level of β-glucosidase activity increases proportionally with the oleuropein and ligustroside content. In the black stage, the phenolic concentration declines and the glucosidase activity is low.

The olive oil phenolic profile depends on the genotype but it is also influenced by the oil extraction process. The phenomenon of malaxation induces the activation of several endogenous fruit enzymes, such as β-glucosidase and esterases, which hydrolyze precursors of oleuropein and ligustroside to produce secoiridoid derivatives.

During the mechanical extraction process, mainly the crushing and malaxing steps, hydrolysis reactions take place catalyzed by the endogenous β-glucosidase and oxidoreductase enzymes of the olive fruits acting on phenolic glycosides.

The malaxation time negatively affects the concentration of major phenolic compounds founds in olive oil, except for oleocanthal and oleacein.

Furthermore, we know from Katsinas et al. (Molecules 2021, Olive Pomace Phenolic Compounds Stability and Safety Evaluation: From Raw Material to Future Ophthalmic Applications, 26, 6002) that the pretreatment of the raw material (olive pomace obtained from the processing of Arbequina variety (2018 crop)) can highly affect the stability of the main phenolic compound. In such experiments, a part of the fresh olive pomace was packed in plastic bags of approximately 1 kg with the use of N₂ (to devoid oxygen) and stored at -20°C for 4 months. The freeze-dried olive pomace was then lyophilized under vacuum (18 kPa), protected from light for 72 h and were also subjected to conventional solid-liquid phenolic extraction by applying a temperature of 70.0°C, a %EtOH of 50.0%, and a solid/liquid ratio (S/L) of 0.5 g RAW olive pomace/mL solvent directly after the pretreatment took place.

Specifically, among all olive tree by-products, olive leaves are thoroughly investigated, and their phenolic content is variant.

As described by Katsinas *et al*., for extracts derived from olive leaves, different drying procedures and storage temperatures have been applied either in liquid hydroalcoholic (80% v/v EtOH) or solid (dried) form for a storage period of 4 weeks. A significant reduction of their total phenolic content (TPC) and antioxidant activity (AA) was observed after drying.

For example, we the know the patent document WO2017122034 which describes a process for increasing the levels of the polyphenols, oleocanthal and oleacein, in olive oil characterized in that for the production of olive oil, olive tree leaves are mixed with the olives, passed through a crusher, a blender, a centrifuge and a separator, to produce the olive oil.

Furthermore, we also know the patent document WO2021121925 which describes a process for the production of synthetic compounds such as oleocanthal or oleacein, starting from oleuropein. The process involves notably enzymatic hydrolysis with b-glucosidase and provides the dialdehyde core of oleocanthal, oleacein and their analogues having the required stereochemistry.

Nevertheless, the need for alternative oleacein's sources is essential, since there is a lack of synthetic oleacein from the market due to its low synthetic yield and huge amounts of olive oil are demanded for oleacein's extraction, making this process unprofitable.

The main aim of the invention is the investigation of alternative plant materials as sources for oleacein recovery and the optimization of the methods and extraction conditions to produce oleacein enriched extracts.

### PROBLEM TO BE SOLVED

The technical problem underlying the present invention is thus the provision of improved or alternative means and methods for increasing the levels of oleacein to obtain an extract enriched in oleacein from alternative natural sources, in particular from olive tree by-products.

### SUMMARY OF THE INVENTION

A first object of the invention is a method for increasing the levels of oleacein in an olive tree leaves extract wherein it comprises the following steps:
- after being collected, olive tree leaves are frozen and pre-treated by freeze drying;
- the freeze-dried olive tree leaves are extracted in a solvent; and
- after filtration, an olive tree leaves extract enriched in oleacein is obtained.].

A second object is an olive tree leaves extract enriched in oleacein obtained according to the method of the invention.

A further object of the invention is a composition comprising the olive tree leaves extract enriched in oleacein according to the method of the invention.

Another object of the invention is the the olive tree leaves extract enriched in oleacein obtained according to the method of the invention for an oral or topical use as a medicament.

### BRIEF DESCRIPTION OF THE DRAWINGS

The invention shall be better understood when reading the following non-restricted description, with reference to the accompanying drawings, wherein:
Figure 1 shows a bar chart comprising comparative data for the extraction yield of olive leaves (OL) dried with 4 different drying methods and extracted with 11 extraction protocols.
Figure 2 shows a bar chart comprising comparative quantification data of oleuropein (OLE) and oleacein (OLEA) content in olive leaves (OL) extracts applying different drying methods and extraction protocols.
Figure 3 shows a bar chart comprising comparative quantification data of oleuropein (OLE) and oleacein (OLEA) content in extracts obtained from olive leaves (OL) dried in ambient conditions and extracted with 10 extraction protocols.
Figure 4 shows a bar chart comprising comparative quantification data of oleuropein (OLE) and oleacein (OLEA) content in olive leaves (OL) extracts applying different drying methods and extraction protocols followed by an additional liquid-liquid extraction step with EtOAc.
Figure 5 illustrates a dendrogram comparing oleacein to 23 other known anti-inflammatory molecules on proteins involved in inflammation.

### DETAILED DESCRIPTION

The present inventors have developed a new, rapid, economical, and eco-friendly method for obtaining oleacein-enriched extracts from alternative natural sources, in particular from olive tree by-products.

During olive oil and table olive production, huge amounts of by-products are produced, such as the olive pomace, the mill wastewaters, the stones, and the leaves. Even if the olive pomace is the main by-product generated and is a mixture of olive fruits after the recovery of olive oil, together with vegetation waters and stones, according to the present invention, we will surprisingly focus on valorization of olive tree leaves.

A first object of the invention is a method for increasing the levels of oleacein in an olive tree leaves extract wherein it comprises the following steps:
- after being collected, olive tree leaves are frozen and pre-treated by freeze drying;
- the freeze-dried olive tree leaves are extracted in a solvent; and
- after filtration, an olive tree leaves extract enriched in oleacein is obtained.

The term olive trees as used herein means any subspecies or varieties of *Olea europaea* originated notably from Mediterranean countries like Spain, Italy, Greece, Morocco, Algeria, Tunisia and France, for example *Olea europaea subsp. cerasiformis, Olea europaea subsp. cuspidata, Olea europaea subsp. guanchica*, *Olea europaea subsp. laperrinei,* and *Olea europaea subsp. Maroccana,* or from olive cultivars Koroneiki, Megaritiki, and Kalamon.

The olive tree leaves collected and used in the method according to the invention are preferably fresh before being frozen and pre-treated by freeze-drying.

The term fresh means an olive tree leave which comprises at least 70% of its total weight in water, *e*.*g*. a water content of 80-90%, conventionally about 85% water, before or after loss through desiccation, especially after being collected directly on trees or after harvest of olive. For example, the olive tree leaves are used in the method according to the invention between a few minutes after being collected to a few hours after, like 1, 3, 5, 12 to 24 hours, and are more preferably used as soon as possible after being collected.

Olive tree leaves are collected directly on olive trees or after harvest of olive, advantageously on the floor after being fall to the ground due to harvesting.

Preferably, the olive tree leaves are not denaturated neither by being crushed nor ground and are used as intact as possible to avoid any enzymatic and non-enzymatic hydrolysis of precursor secoiridoid glucosides before being pre-treated by freeze drying.

The collected olive tree leaves are frozen, preferably at least at -20°C, for example -20°C, -30°C, -40°C, -50°C, -60°C, -70°C, -80°C, at -80°C, preferably -20°C or - 80°C, during at least 12h, preferably 24h, before being pre-treated by freeze drying.

The pre-treatment by freeze drying is an essential step according to the method of the invention, compared to natural, microwave or oven drying.

Freeze drying is preferably performed at a temperature of between -35°C to - 80°C, preferably between -35°C to -50°C, more preferably -47°C, preferably under vacuum at 0.2 mBar, and preferably for 5 to 7 days.

After pre-treatment, the freeze-dried olive tree leaves are preferably ground before being extracted.

According to a preferred embodiment, the freeze-dried olive leaves are ground in a cutting mill apparatus, resulting to a plant material powder with the 95% of particles diameter ranging from 250 µm to 80 µm.

The freeze-dried olive leaves, preferably ground, are then extracted in a solvent.

The extraction can be in batch or continuous and dynamic (under stirring) or static, preferably dynamic.

According to another embodiment, alternatively to a solvent extraction, conventional extraction or alternatives processes like supercritical fluid extraction (SFE), microwave assisted extraction (MAE), ultrasound assisted extraction (UAE), or extraction assisted with ultrasound and microwave (UMAE) can also be used.

The extraction procedures are performed in any conventional means, like an extractor, at the adjustment temperature, preferably under stirring.

The solvent is preferably an organic solution chosen from methanol, ethanol, ethyl acetate, dimethylcarbonate, preferably an ethanol or methanol solution, or an aqueous solution modified or not with a base, for example an acidic aqueous solution of pH 5 with Na2SO4 or a basic aqueous solution of pH 8 with NaHCO3.

The solvent can also be chosen from eutectic solvents, for example Deep Eutectic Solvents (DES) and Natural deep eutectic solvents (NADES).

According to a preferred embodiment, the freeze-dried olive tree leaves, preferably ground, are extracted in ethanol or methanol, preferably ethanol, preferably at 60°C for 60 min.

According to another embodiment, the freeze-dried olive tree leaves, preferably ground, are extracted in an aqueous solution at different pH values of 5 for example with Na2SO4 at 0.1M, 7, or 8 for example with NaHCO3 at 0.1 M and at different temperatures of 4, 25, 50 and 85°C for 60 minutes.

According to another preferred embodiment, the freeze-dried olive tree leaves, preferably ground, are extracted in an aqueous solution at pH 7 and at 85°C.

According the method of the invention, the freeze-dried olive tree leaves, preferably ground, are extracted in a solvent wherein the ratio freeze-dried olive tree leaves : solvent is between 1:20 and 1:5 preferably 1:10.

The extracted freeze-dried olive tree leaves is then filtrated.

Filtration is performed using any conventional means. Preferably, the filtration is performed under vacuum or under pressure.

According to the method of the invention, after filtration, an olive tree leaves extract enriched in oleacein is obtained.

Surprisingly, the olive tree leaves extract obtained according to the method of the invention is enriched in oleacein, wherein the oleacein content is preferably more than 5% w/w, more preferably more than 10% w/w, even more preferably more than 13% w/w.

Advantageously, the solvent is evaporated.

According to another embodiment, after filtration and before recovering of the olive tree leaves extract enriched in oleacein, a further liquid-liquid extraction step of the olive tree leaves extract is performed with ethyl acetate (EtOAc).

For example, the aqueous phase is subsequently extracted at least one, preferably two, bath(s) of ethyl acetate.

The organic phase is then dried on a drying agent and the solvent is evaporate to dryness under water pump vacuum and at a temperature that does not exceed 45°C.

Then the extract can be spray dried with or without a carrier like maltodextrin, arabic gum, ... or other carrier to help the spray drying.

According to another embodiment of the invention, in addition to the olive tree leaves, olive fruits are added to the olive tree leaves before being frozen and pre-treated by freeze drying.

A second object of the invention is an olive tree leaves extract enriched in oleacein obtained according to method of the invention, preferably wherein the oleacein content is more than 5% w/w, more preferably more than 10% w/w, even more preferably more than 13% w/w.

A further object of the invention is a composition comprising the olive tree leaves extract enriched in oleacein according to the invention, preferably wherein the oleacein content is more than 5% w/w, more preferably more than 10% w/w, even more preferably more than 13% w/w.

Such composition comprising, in a physiologically acceptable medium, an olive tree leaves extract obtainable by the method according to the invention.

A physiologically acceptable medium means a medium suitable for use in contact with human and animal cells, in particular epidermal cells, without toxicity, irritation, undue allergic response and the like, and commensurate with a reasonable benefit/risk ratio.

Such a physiologically acceptable medium may comprise excipients and/or adjuvants known and used in the cosmetic and pharmacological fields.

For instance, the physiologically acceptable medium may include, but is not limited to, one or more of the following agents: solvents, emulsifiers, suspending agents, decomposers, binding agents, chelating agents, stabilizing agents, diluents, antioxidants, gelling agents, preservatives, lubricants, absorption delaying agents, skin-penetrating agents, liposomes, coloring materials, odor absorbers or pigments and a mixture thereof.

The amounts of the different constituents of the compositions according to the invention are those conventionally used in the field under consideration.

The person skilled in the art will take care to choose the physiologically acceptable medium in such a way that it does not adversely affect the interesting properties of the extract and the composition according to the invention.

The composition according to the present invention may preferably be administered orally or topically.

In some embodiments, the composition of the invention is formulated for oral administration to a patient.

Compositions of the invention may be orally administered in any orally acceptable dosage form including, but not limited to, capsules, tablets, aqueous suspensions or solutions. If desired, certain sweetening, flavoring or coloring agents may be added.

In some other embodiments, the composition of the invention is formulated for topical administration to a patient.

The term "topically administrable composition," a "topical composition," or a "topical formulation," as used herein, means any formulation or composition which is pharmaceutically acceptable for topical delivery of the olive tree leaves extract according to embodiments of the invention, which can be applied on the skin and/or the mucous membranes.

The topical compositions according to the invention are suited for treating the skin. They can be in liquid, more or less fluid, pasty or solid form, and more particularly in the form of salves, ointments, emulsions, creams, milks, pomades, powders, impregnated pads, syndets, towelettes, solutions, gels, sprays or aerosols, foams, suspensions, lotions, sticks, shampoos or washing bases. Preferably, the composition used in the present invention is in the form of an emulsion, of a cream, of a lotion type, of a gel, or of a solution, and, more preferably in the form of an emulsion. For example, it may be in the form of an optionally gelled, oily solution, an optionally two-phase dispersion of the lotion type, an emulsion obtained by dispersion of a fatty phase in an aqueous phase (O/W) or vice versa (W/O), or a triple emulsion (W/O/W or O/W/O) or a vesicular dispersion of ionic and/or nonionic type. They can also be in the form of suspensions of microspheres or nanospheres or of lipid or polymeric vesicles or of polymeric patches and of hydrogels for controlled release. These compositions for topical application can be in anhydrous form, in aqueous form, or in the form of an emulsion.

The pH of the topical compositions of the invention is preferably within a physiologically acceptable pH, e.g., within a range of about 5 to about 7, preferably within the range of about 5 to 6.

Another object of the invention is the the olive tree leaves extract enriched in oleacein obtained according to the invention for an oral or topical use as a medicament, preferably for use for the treatment of inflammatory diseases or cardiopathic diseases, more preferably for the treatment of inflammatory diseases, particularly inflammatory articular diseases which encompass a spectrum of conditions that range from acute forms of septic or sterile arthritis to chronic, often polyarticular conditions; for example of inflammatory articular diseases, we can cite the two most prevalent forms of chronic immune-mediated inflammatory disease of the musculoskeletal system: rheumatoid arthritis and spondyloarthritis. Other types of inflammatory arthritis could be treated and are chosen from:
- psoriatic arthritis, in which scaly spots may appear on the skin, along with swelling in multiple joints and back stiffness, and at times, diffuse swelling of a finger or toe, giving the digit a "sausage-like" appearance;
- gout and pseudogout, in which needle-like crystals of uric acid or rhomboid-shaped crystals of calcium pyrophosphate, respectively, form in cartilage and are released into the joint fluid, producing painful effects. Gout is most common in the first toe, where it meets the foot (the metatarso-phalangeal joint), but may also involve a number of other joints, such as the midfoot, ankle, knee and bursa of the elbow. Pseudogout occurs, generally, in an older population, and especially involves the ankles, knees, wrists and shoulders;
- ankylosing spondylitis and related conditions affect the spine as well as peripheral joints. These conditions are associated with severe morning stiffness in the spine. In certain cases, other organs such as the eyes and heart may be affected;
- Parvovirus arthritis is a contagious, viral form of arthritis that can cause flu-like symptoms and rashes along with usual IA symptoms. Children with "fifth disease" due to parvovirus will often have a "slapped cheek" presentation of their rash, while their parents can present with a severe systemic inflammatory arthritis looking much like rheumatoid arthritis. Although parvovirus arthritis commonly resolves on its own within a few weeks, 10% of patients may experience ongoing symptoms;
- Lyme disease, caused by a spirochete (bacteria) contracted through the bite of an infected deer tick, produces IA symptoms in its later stages but can include early symptoms such as rashes, fatigue, chills, fever, headache, and a stiff, aching neck. Early antibiotic treatment is critical here. About 50% of patients are aware of a tick bite and/or the typical "target lesion" suggesting Lyme disease, but many cases need to be diagnosed based on a patient having been in an area known to be infected with the Lyme organism, presenting with symptoms consistent with Lyme disease. Blood testing is very useful once arthritis develops, but may be falsely negative very early in Lyme disease; and
- systemic lupus erythematosus (commonly called lupus or SLE) is an inflammatory condition which can involve essentially all systems of the body. Arthritis is one of the single most common ways for lupus to present, and lupus arthritis appears similar to the early stages of rheumatoid arthritis. Clues to lupus include a sun-sensitive skin rash, mouth sores, hair loss, chest pain with a deep breath, and a positive ANA blood test.

Advantageously, oleacein has some anti-inflammatory properties.

Oleacein decreases cyclooxygenase 2 activity, thereby reducing inflammation and is also thought to be responsible for an anti-sclerotic effect. Oleacein decreases inflammation by the inhibition of the TNF a induced production of the pro-inflammatory gene CCL2 and inhibition of CCL2 transcription.

Oleacein also exhibits a down regulatory effect on the expression of adhesion molecules VCAM-1, ICAM-1 and E selectin.

Oleacein enhances anti-inflammatory activity by stimulating the expression of CD163, an anti-inflammatory gene, increasing the secretion of two anti-inflammatory factors, IL10 and heme oxygenase 1.

Oleacein also inhibits arachidonate 5 lipoxygenase, which is responsible for the first steps of the biosynthesis of pro inflammatory leukotrienes.

Oleacein could have a protective role in human blood cells against oxidative induced hemolysis.

It reduces H2O2 induced DNA damage in human blood mononuclear cells and also protects LDL from oxidation.

Oleacein is also known to be a scavenger of free radicals. Its remarkable antioxidant action is due to the decrease in myeloperoxidase (MPO) release by human neutrophils.

Oleacein is a stronger inhibitor of neutrophil's oxidative burst, myeloperoxidase (MPO) release and is a more potent scavenger of HOCI than is oleuropein.

MPO plays a key role in atherogenesis by affecting lipid peroxidation and the generation of reactive nitrogen species, which in turn convert low density lipoprotein into an atherogenic form.

Stimulated neutrophils release myeloperoxidase, which is the enzyme capable of catalyzing the formation of HOCI, a highly reactive species responsible for oxidation and chlorination reactions.

Both oleacein and oleuropein significantly reduce myeloperoxidase release from polymorphonuclear cells (PMNs).

The inhibitory effect of oleacein is dependent on the concentration. Oleacein is a more than twice stronger inhibitor than oleuropein.

In addition to the antioxidant action, Oleacein shows anti-inflammatory effects through the stimulation of macrophage activity (increase in the expression of CD163 macrophage receptors).

It also decreases the adhesion of monocytes to vascular endothelial cells by inhibiting the expression of adhesion molecules E selectin, vascular cell adhesion molecules 1 and intercellular adhesion molecule 1.

Advantageously, oleacein has also some cardio-protective effects.

Oleacein may play a special role in decreasing the progression of atherosclerosis.

It is known to prevent oxidation, inhibit neutrophil adhesion and reduce blood pressure.

The protective effect of oleacein against cardiovascular diseases, mainly atherosclerosis, is due to different actions:
- Reduction of oxidation;
- Lowering of hypertension through the inhibition of the angiotensin converting enzyme;
- Suppressing the in vitro production of superoxide and LPS induced NO.

Oleacein reduces neutrophil release by myeloperoxidase, which is responsible for lipid peroxidation and generates reactive nitrogen species.

Oleacein reduces the level of low density lipoprotein in the atherogenic form.

Oleacein is also known to inhibit angiotensin converting enzyme (ACE) activity in vitro (Hansen et al.).

Oleacein may play a much more important role against ROS/RNS-induced injury of human biomolecules than does oleuropein and it is for this reason, it contributes to the cardiovascular health benefits notably of olive oil in the Mediterranean diet.

### EXAMPLES

### EXAMPLE 1: Comparative data for the extraction yield of olive leaves dried with 4 different drying methods and extracted with 11 extraction protocols

For this experimental stage, olive leaves collected from Figalia, Peloponnese, Greece, in May 2020 were used.

100g of fresh olive leaves of the Olea europaea L. species, including all subspecies and cultivars, were dried with 4 different drying protocols and specifically:
(a) naturally, at room temperature and to the exclusion of light for one week,
(b) in a laboratory oven at 140°C for 26 minutes,
(c) by microwave radiation (700 W) for 5 minutes and
(d) in a freeze-drying apparatus at a temperature of between -35°C to -50°C, preferably -47°C, under vacuum (lyophilization at 0.2 mBar), for 5 to 7 days, the olive leaves having previously been frozen at -80°C for 24 hours.

Afterwards, the dried olive leaves were ground in cutting mill apparatus, resulting to a plant material powder with the 95% of particles diameter ranging from 250µm to 80µm. The dried ground leaves were then extracted either with methanol or with water at different pH values and at different temperatures.

In all cases the ratio of dried plant material/extraction solvent was 1/10 and the extraction time was 60 minutes.

The extraction procedures were performed in a silica oil bath, at the adjustment temperature, under stirring.

### Alcoholic extraction

The alcoholic extraction procedure of olive leaves was performed according to a modified protocol of European Pharmacopoeia (Oleae folium 01/2009:1878 - 8th Edition).

Specifically, 10 g of the dried ground leaves was mixed with 100 ml methanol (MeOH) and the extraction process was achieved in a silica oil bath, at 60°C, for 60 minutes, under stirring. The methanol extracts were then filtered under vacuum and the solvent was evaporated by a Rotary evaporation apparatus. A syrupy product is thus recuperated, which was rich in oleuropein (content 20-35%), but without the oleacein component to be detected, except in the case of freeze-drying olive leaves where the oleacein content of the extract was 15%.

### Aqueous extraction

Aqueous extractions of olive leaves were performed applying the following extraction protocol.

10 g of the dried ground leaves were mixed with either 100 ml of 0.1 M sodium sulfate (Na2SO4, 0.1M) (pH 5.0), or 100 mL of 0.1 M sodium bicarbonate (NaHCO3 0.1 M) (pH 8.0), or with distilled water.

The extraction process was performed in a silica oil bath, at four different temperatures 4, 25, 50 and 85°C, for 60 minutes, under stirring.

Afterwards, the solid residues are filtered, and the aqueous phases are subsequently extracted with 2x100 ml ethyl acetate. The organic phase is then dried on a drying agent and the solvent is evaporate to dryness underwater pump vacuum and at a temperature that does not exceed 45°C.

A syrupy product is thus recuperated, which is chlorophyll free and enriched either in oleuropein (content 2-85%), or in oleacein (content 0-58%).

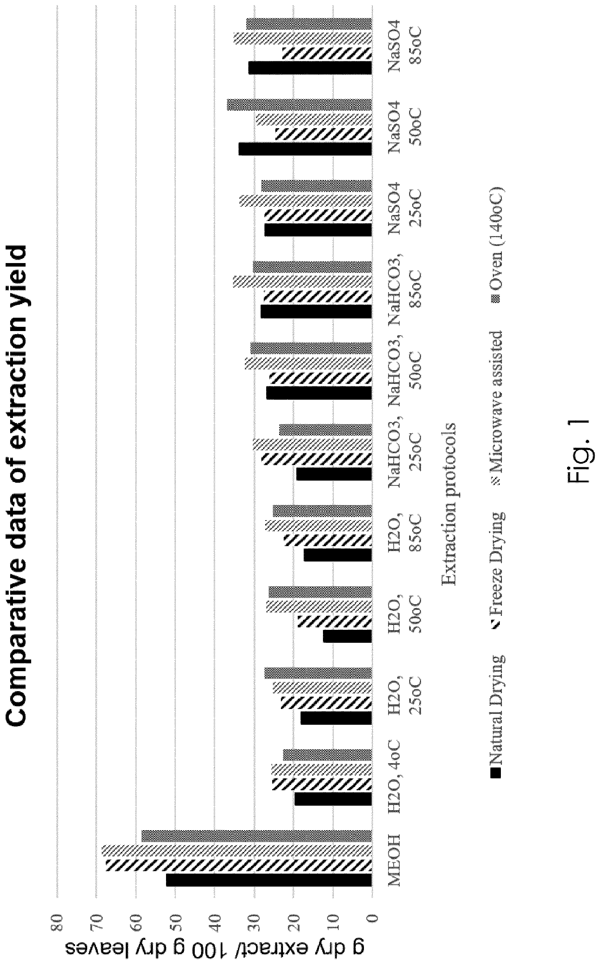

As shown in Figure 1, the bar chart represents the comparative data of the extraction yield of all the extracts obtained from olive leaves dried with the aforementioned drying procedures and the extraction protocols.

It is obvious that olive leaves extracted with methanol yield noteworthy higher amounts of dry extracts compared to water extraction. Microwave and oven (140°C) assisted dried olive leaves yield even 120% higher amounts of extracts compared to leaves dried in ambient conditions. This is probably due to the damage caused in membrane cells during microwave and oven (140°C) assisted drying procedures, inducing the release of polar compounds from the cellular organelles, facilitating their extraction into solvent and increasing the extraction yield.

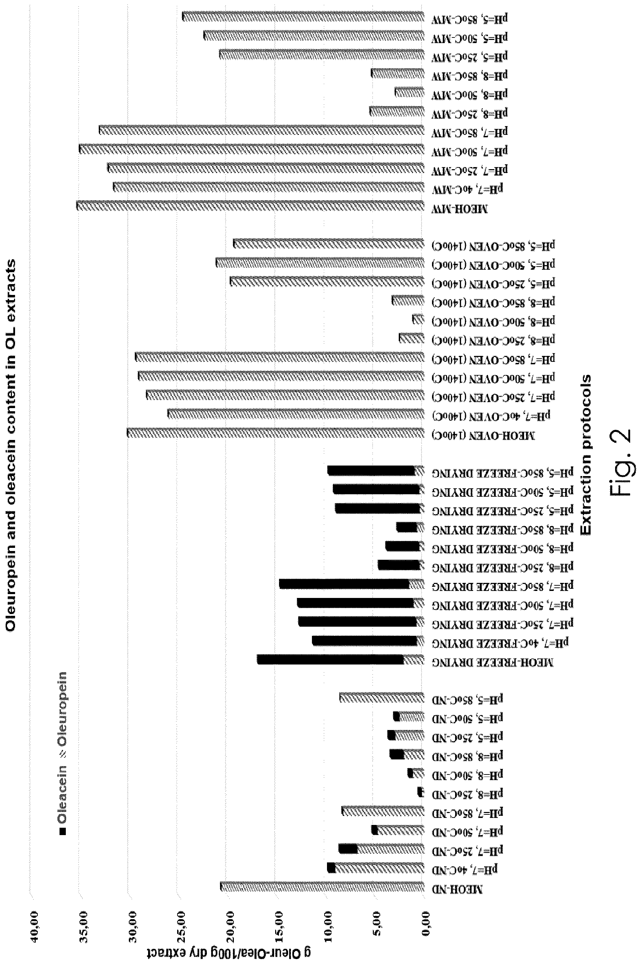

As shown in Figure 2, the bar chart represents the comparative data for the quantification of Oleuropein (OLE) and Oleacein (OLEA) in olive leaves dried with 4 drying methods and extracted with 11 different extraction protocols.

Microwave assisted and oven (140°C) dried olive leaves produce extracts with the highest OLE content regardless of the extraction protocol. Specifically, the extracts obtained from olive leaves dried in a microwave apparatus and treated with methanol at 60°C or water at 50°C, contained 35.4 g OLE/100 g dry extract and 35.1 g OLE/100 g dry extract respectively.

Freeze dried olive leaves produced OLEA enriched extracts regardless of the extraction protocol. In particular, freeze-dried olive leaves extracted with methanol at 60°C or water at 85°C, contained 14.8 g OLEA/100 g dry extract and 13.1 g OLEA/100 g dry extract respectively.

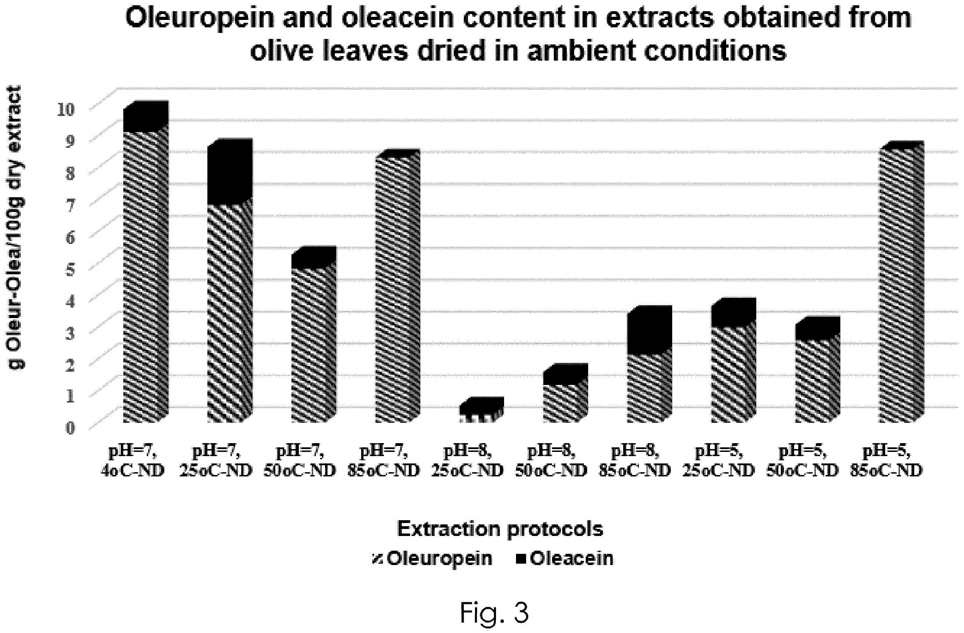

Amounts of OLEA (but less than 10 g OLEA/100 g dry extract) were also quantified in olive leaves dried in ambient conditions and extracted with water; however, the OLEA content depends on the type of solvent, pH value and extraction temperature as shown in Figure 3.

For example, extraction of ambient dried olive leaves with MeOH does not recover oleacein.

OLEA was quantified under LOQ (Limit of Quantification) and LOD (Limit of Detection) in methanol extracts of naturally dried olive leaves.

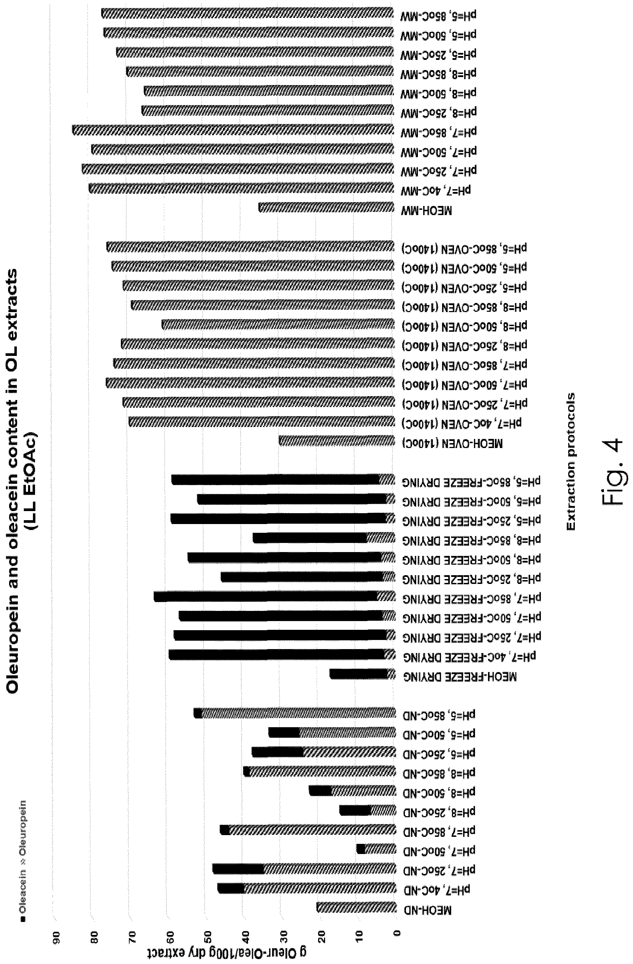

Furthermore, the aqueous extracts obtained with all the above procedures were submitted to an additional liquid - liquid extraction step with EtOAc (LL EtOAc)). The concentrations levels of OLE and OLEA increased significantly in the organic phase (EtOAc) extracts, as shown in Figure 4.

Water extraction of olive leaves dried by wicrowave at pH=7 and 85°C, followed by an additional liquid - liquid extraction step with EtOAc led to a yield of 84,4 g OLE per 100g extract.

Water extraction of Freeze-Dried olive leaves at pH=7 and 85°C led to a yield of 58,6 g OLEA per 100g extract, after LL EtOAc extraction.

Finally, water extraction of ambient dried olive leaves at pH=5 and 25°C followed by an additional LL extraction step with EtOAc, led to OLEA-enriched extracts containing up to 13,2 g OLEA/ 100g dry extract.

For the comprehension of the above results, this study suggests that enzymes perform key roles and are related strongly with the phenolic composition of olive leaves extracts. Comparing all drying methods, microwave, and oven (140°C) dried olive leaves extracts have the highest amounts of OLE. A possible explanation to this is that the high intensity of microwave radiation and the big amounts of thermal energy applied in the oven at 140°C inactivate the endogenous enzymes (b-glucosidase, esterase, peroxidase, and polyphenoloxidase (PPO) and do not favor oleuropein enzymatic biotransformation into oleacein, leading to the significant levels of oleuropein in extracts.

However, during the thawing of the frozen olive leaves, the cell membranes of the plant material are broken down, having as result the enzymes (β-glucosidase, esterase, polyphenyl oxidase) and polyphenols, which were originally located in different cellular compartments (chloroplasts and cytosol respectively), interact with each other and thus the enzymatic reactions of oxidation, degradation, and transformation of Oleuropein in secoiridoid derivatives are favored. Specifically, when the leaves are thawed in the absence of atmospheric oxygen, as in the case of freeze-drying procedure, the oxidizing enzymes (polyphenyloxidase, peroxidase) of olive leaves are inactivated, while β-glucosidase and esterase maintain their activity, catalyzing the conversion of oleuropein to oleacein.

In natural drying conditions, the dehydration of the cells takes place through capillary phenomena and diffusion mechanisms, so the moisture is removed from the cell without rupturing its cell membrane. Thus, when the extraction of naturally dried olive leaves takes place in the presence of water, the activity of the enzymes of the leaves increases, resulting to enzymatic biotransformation of oleuropein into oleacein. The high content of oleacein in the extracts of olive leaves, produced in neutral or slightly acidic conditions and at relatively low temperatures, is probably due to the activity of the enzymes, esterase and β-glucosidase, where they have the maximum activity at pH = 5 and at low temperatures. The above enzymatic reactions do not appear to be favored when methanol is used as the extraction solvent. The excess of methanol in the extract means prevents the hydrolysis of methyl ester and the formation of demethylated oleuropein, which is a prerequisite for the conversion of oleuropein to oleacein. Indeed, hydrolysis of methyl ester is a bidirectional chemical reaction where increasing the concentration of methanol in the products, the position of the chemical equilibrium shifts to the methyl ester side and thus does not favor the formation of demethylated oleuropein.

### Conclusion

The most efficient drying methods for obtaining OLE-enriched extract from olive leaves are those of the microwave drying, and the oven drying at high temperatures. Extracts obtained from microwave and Oven-dried olive leaves showed the highest OLE recovery, compared to the natural dried ones.

Extraction of ambient dried olive leaves with water at neutral or mild acetic pH values at room temperature, followed by an additional liquid - liquid extraction step with EtOAc is a fast, profitable, and eco-friendly method for the production of oleacein enriched extracts.

Surprisingly, the freeze drying of olive leaves method leads to the higher production of oleacein enriched extracts regardless of the extraction protocol applied.

### EXAMPLE 2: in silico results about oleacein anti-inflammatory properties

An original *in silico* approach has been performed to compare oleacein to 23 other known anti-inflammatory molecules on proteins involved in inflammation.

With regard to the protocol for simulating the activity potential of the molecule: the simulation is performed in a comparative way with other molecules known for their efficacy as anti-inflammatory drugs which are thus used as controls.
The simulation is performed in two different ways:
- each molecule is evaluated by a docking method on proteins known to be involved in inflammation.
This assessment is quantified by a score.
We thus obtain for each molecule a set of scores that represent the "fingerprint" of docking of the molecule. It is thus possible to compare the fingerprint of the molecule tested with the fingerprints of the control molecules.
- The molecular structure "fingerprint" is then traced for the molecule tested and for each control molecule.
We then compare the proximity between the fingerprint of the molecule to be tested with the fingerprints of the control molecules.
Once these comparisons are completed, we are able to predict the pharmacological potential of the molecule tested and to place it in comparison with the control molecules. We are also able to obtain a therapeutically effective dose for oleacein.

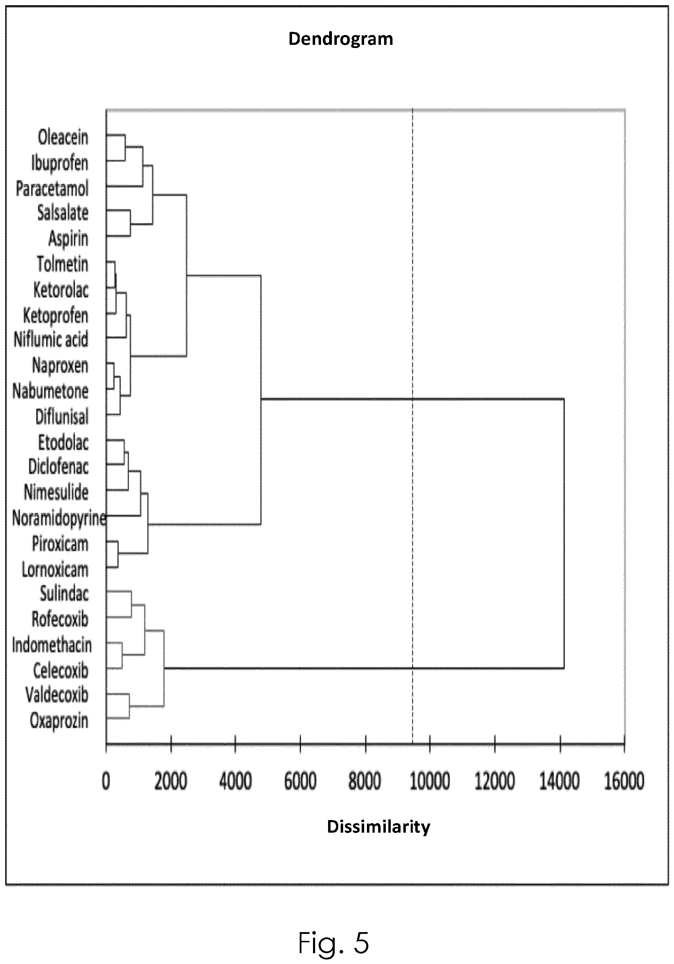

Based on the results, as illustrated by Figure 5, oleacein should behave as a salicylated derivative without the expected digestive side effects, and it should have a similar mode of action and an intensity of action close to aspirin.

Furthermore, according to these in silico results obtained, the predicted effective dose of oleacein should be between 1300 mg and 2400 mg.

## Claims

1. Method for increasing the levels of oleacein in an olive tree leaves extract wherein it comprises the following steps:
- after being collected, olive tree leaves are frozen and pre-treated by freeze drying;
- the freeze-dried olive tree leaves are extracted in a solvent; and
- after filtration, an olive tree leaves extract enriched in oleacein is obtained.

2. The method according to claim 1, wherein the olive tree leaves are fresh before being frozen and pre-treated by freeze-drying.

3. The method according any of claim 1 or 2, wherein the collected olive tree leaves are frozen at least at -20°C during at least 12h before being pre-treated by freeze drying.

4. The method according to any one of the preceding claims, wherein freeze drying is performed at a temperature of between -35°C to -80°C, preferably between - 35°C to -50°C.

5. The method according to any one of the preceding claims, wherein the freeze-dried olive tree leaves are ground before being extracted.

6. The method according to any one of the preceding claims, wherein the solvent is an organic solution chosen from methanol, ethanol, ethyl acetate, dimethylcarbonate or an aqueous solution modified or not with a base.

7. The method according to claim 6, wherein the freeze-dried olive tree leaves are extracted in ethanol or methanol, preferably at 60°C for 60 min.

8. The method according to claim 6, wherein the freeze-dried olive tree leaves are extracted in an aqueous solution at different pH values of 5, 7, 8 and at different temperatures of 4, 25, 50 and 85°C for 60 minutes.

9. The method according to claim 8, wherein the freeze-dried olive tree leaves are extracted in an aqueous solution at pH 7 and at 85°C.

10. The method according to any one of the preceding claims, wherein the ratio freeze-dried olive tree leaves : solvent is between 1:20 and 1:5, preferably 1:10.

11. An olive tree leaves extract enriched in oleacein obtained according to any of claims 1 to 10.

12. The olive tree leaves extract according to claim 11, wherein the oleacein content is more than 5% w/w, preferably more than 10% w/w, more preferably more than 13% w/w.

13. A composition comprising the olive tree leaves extract enriched in oleacein according to claim 11 or 12.

14. The olive tree leaves extract enriched in oleacein according to claim 11 or 12 for an oral or topical use as a medicament.
